## Europäisches Patentamt
## European Patent Office
## Office européen des brevets

(11) Veröffentlichungsnummer: **0 200 919**
**B1**

(12) # EUROPÄISCHE PATENTSCHRIFT

(45) Veröffentlichungstag der Patentschrift:
28.06.89

(51) Int. Cl.⁴: **A 61 M 25/00**

(21) Anmeldenummer: 86104521.9

(22) Anmeldetag: 02.04.86

(54) **Führungsteil.**

(30) Priorität: 04.05.85 DE 3516052

(43) Veröffentlichungstag der Anmeldung:
12.11.86 Patentblatt 86/46

(45) Bekanntmachung des Hinweises auf die Patenterteilung: 28.06.89 Patentblatt 89/26

(84) Benannte Vertragsstaaten:
AT BE CH DE FR GB IT LI LU NL SE

(56) Entgegenhaltungen:
EP–A– 0 132 694
FR–A– 564 832
US–A– 3 528 406
US–A– 3 612 058
US–A– 3 749 086
US–A– 3 789 841
US–A– 4 080 706

(73) Patentinhaber: ANGIOMED Aktiengesellschaft
Eisenbahnstrasse 36
D-7500 Karlsruhe 41 (DE)

(72) Erfinder: Schüller,Jörg, Prof. Dr.
Moltkestrasse 8
D-2400 Lübeck (DE)

(74) Vertreter: Dr.-Ing. Hans Lichti Dipl.-Ing. Heiner Lichti
Dipl.-Phys. Dr. Jost Lempert
Postfach 41 07 60 Durlacher Strasse 31
D-7500 Karlsruhe 41 (DE)

## Beschreibung

Die Erfindung bezieht sich auf ein Führungsteil für einen in einen Körperhohlraum einzuführenden langgestreckten Gegenstand, wie einen Katheterschlauch, einen rohrförmigen Dilatator od. dgl., mit einem drahtförmigen Teil, der einen flexibel-biegbaren Strangkörper aufweist, der als Litze aus verseilt umeinandergewickelten Drähten ausgebildet ist.

Die Punktion von Körperhohlräumen, z. B. Nierenbecken, Gallengang, Blase, Venen und Arterien geschieht sehr häufig zum nachträglichen Einlegen eines Katheters oder einer Sonde. Da der Durchmesser des einzuführenden Katheters oder der Sonde häufig größer ist als der Durchmesser der zur Punktion benutzten Stahlkanüle, ist in diesen Fällen eine Dilatation des Stichkanals erforderlich. Zu diesem Zweck werden verhältnismäßig steife Dilatatoren, z. B. aus Polypropylen, verwendet. Um nach Zurückziehen der Stahlkanüle das Punktionsloch in der Wand des Hohlraumes und den Stichkanal wiederzufinden, wird nach der Punktion durch die Stahlkanüle ein Führungsteil der eingangs erwähnten Art in den Körperhohlraum eingeschoben. Dieser Führungsteil bleibt mit seiner weich-flexiblen Spitze in dem Körperhohlraum liegen, während sein langer drahtförmiger Teil aus der Haut herausragt. Sodann wird die Stahlkanüle über den drahtförmigen Teil zurückgezogen. Anschließend wird der Dilatator od. dgl. auf den drahtförmigen Teil aufgeschoben und von diesem durch Haut, Muskeln und Fascien hindurch in den Körperhohlraum geführt. Anschließend wird der drahtförmige Führungsteil aus dem Körperhohlraum entfernt.

Bei den aus der Praxis bekannten Führungsteilen ist der drahtförmige Teil als Stab oder Rohr und damit verhältnismäßig steif ausgebildet. Da der steife Teil eine Länge von 50 bis 80 cm aufweist, ist eine sehr lange Verpackung nötig, die sowohl für den Versand als auch bei der Aufbewahrung derartiger Führungsteile unpraktisch ist. Außerdem ragt während der Anwendung der steife Teil aus dem Körper des Patienten in den Raum. Hierdurch wird die Handhabung bei der Einführung eines Katheterschlauches, Dilatators od. dgl. für den Anwender erschwert.

Weiterhin sind auch mit weich-flexibler Spitze versehene Führungsteile bekannt, deren drahtförmiger Teil auf seiner ganzen Länge aus einem mit geringer Steigung schraubenförmig enggewickelten Draht besteht, der eine Seele in Form eines den Hohlraum ausfüllenden Drahtes besitzt. Diese Führungsteile sind jedoch manchmal zu weich für das benötigte Einsatzgebiet.

Ein gattungsgemäßes Führungsteil mit einem drahtförmigen Teil in Form einer Litze aus verseilt umeinandergewickelten Einzeldrähten und am distalen Ende angeschweißter Plombe ist aus der EP-A-132 694 bekannt. Nachteilig ist die hier über die gesamte Länge gleichmäßige Stärke des Führungsteils, die über den größten mittleren Teil zu weich ist, so daß hier ein Knicken oder Auslenken erfolgen kann, wenn die Spitze auf einen Widerstand stößt, andererseits im Spitzenbereich zu steif ist, was zu Belastungen oder Verletzungen führen kann.

Weiterhin ist aus der US-A-3 528 406 ein flexibles Führungsteil mit einem schraubenförmigen Außenmantel bekannt, in dessen Innerem Versteifungsdrähte angeordnet sind, die sich zur distalen Spitze hin reduzieren oder verjüngen, um deren Flexibilität zu erhöhen. Hierdurch wird im Spitzenbereich eine größere Weichheit erreicht, die Steifheit über den größten Teil des einzuführenden Führungsteils, das Längen von mehreren zig Zentimetern aufweisen soll, ist aber auch hier nicht ausreichend.

Der Erfindung liegt daher die Aufgabe zugrunde, einen Führungsteil der eingangs genannten Art derart auszubilden, daß eine erleichterte Anwendung durch die Möglichkeit eines stabilen schienenartigen Einführens sowie flexible Biegsamkeit des extrakorporalen langen proximalen Endes bei vertretbarer Verpackungslänge erreicht wird.

Diese Aufgabe wird dadurch gelöst, daß zwischen dem flexibel-biegbaren Strangkörper und einer weich-flexiblen Spitze ein relativ steifer Schaft angeordnet ist.

Bei einem solchen Führungsteil erhält der drahtförmige Teil mittlere Steifigkeit, die einerseits ausreicht, um Dilatatoren od. dgl. durch Haut, Muskeln und Fascien hindurchzuführen und die andererseits weich genug ist, um das extrakorporale Ende des Führungsteils flexibel biegen zu können, so daß es bei der Handhabung nicht stört. Die mittlere Steifigkeit des flexibel-biegbaren Strangkörpers ermöglicht außerdem eine verkürzte Verpackung des Führungsteils, weil der flexibel-biegbare Strangkörper sich zusammenrollen oder umbiegen läßt. Der relativ steife Schaft kann aus einem Rohr oder einem massiven Runddraht bestehen, wodurch sich unterschiedliche Steifigkeiten des Schaftes erzielen lassen. Der relativ steife Schaft zwischen der weich-flexiblen Spitze und dem flexibel-biegbaren Strangkörper braucht nur so lang zu sein, daß er durch Haut, Muskeln und Fascien hindurch die Führung der Dilatatoren od. dgl. übernehmen kann, während das über den Körper des Patienten nach außen vorstehende Stück des drahtförmigen Teils aus dem flexibel-biegbaren Strangkörper besteht, der den Anwender bei der Handhabung nicht stört. Der flexibel-biegbare Strangkörper ist vorteilhafterweise länger als der relativ steife Schaft. Dies hat zur Folge, daß das extrakorporale Ende beim Aufschieben eines Dilatators o. dgl. gut handhabbar ist. Da der relativ steife Schaft recht kurz sein kann und der flexibel-biegbare Strangkörper sich zusammenrollen oder umbiegen läßt, ergibt sich eine günstige verkürzte Verpackungslänge. Es ist vorteilhaft, daß der relativ steife Schaft etwa 30 cm lang, der flexibel-biegbare Strangkörper mindestens etwa 50 cm lang und die Spitze etwa 6 cm lang sind. Alternativ kann für besondere Einsatzzwecke der relativ steife Schaft etwa 1 bis 5 cm lang, der flexibel-biegbare Strangkörper etwa 70 bis 80 cm lang und die Spitze etwa 2 bis 5 cm lang sein.

Die Drähte der Litze des flexibel-biegbaren Strangkörpers sind in mindestens einer Lage konzentrisch um eine Seele gewickelt. Durch gezielte Auswahl der Stärke der Seele und der Drähte sowie der Art der Verseilung kann die Steifigkeit des flexibel-biegbaren Strangkörpers beliebig eingestellt werden, so daß für unterschiedliche medizinische Anwendungsgebiete speziell geeignete Führungsteile herstellbar sind.

Zweckmäßigerweise besteht die weich-flexible Spitze aus einem dünnen Draht, der schraubenförmig mit sehr geringer Steigung eng gewickelt ist und im Inneren einen Fangdraht enthält, der einenends mit einem äußeren Spitzenkörper und anderenends mit dem drahtförmigen Teil verbunden ist. Die Durchmesser der koaxial aufeinanderfolgenden Abschnitte des Führungsteils sind im wesentlichen gleich und die Befestigungsstellen werden absatzlos ausgeführt, so daß die Anschlüsse verdickungsfrei ineinander übergehen und der Vorschub eines zu führenden Dilatators o. dgl. nicht behindert wird.

Der drahtförmige Teil, der aus dem flexibelbiegbaren Strangkörper und dem relativ steifen Schaft besteht, kann aus rostfreiem Metall hergestellt und ggf. mit einer Kunststoffummantelung umgeben sein. Auch die Spitze besteht zweckmäßigerweise aus rostfreiem Metall. Die Kunststoffummantelung verbessert die Glätte der Oberfläche des Führungsteils.

Alternativ kann der drahtförmige Teil aus Kunststoff hergestellt sein, und zwar sowohl der flexibel-biegbare Strangkörper als auch der ggf. vorhandene relativ steife Schaft.

In der Zeichnung sind Ausführungsbeispiele der Erfindung schematisch dargestellt.

Es zeigen :

Fig. 1 eine teilweise geschnittene Draufsicht auf einen Führungsteil mit relativ steifem Schaft zwischen Spitze und Strangkörper, und

Fig. 2, 3 und 4 Querschnitte längs der Linien II-II ; III-III ; IV-IV in Fig. 1.

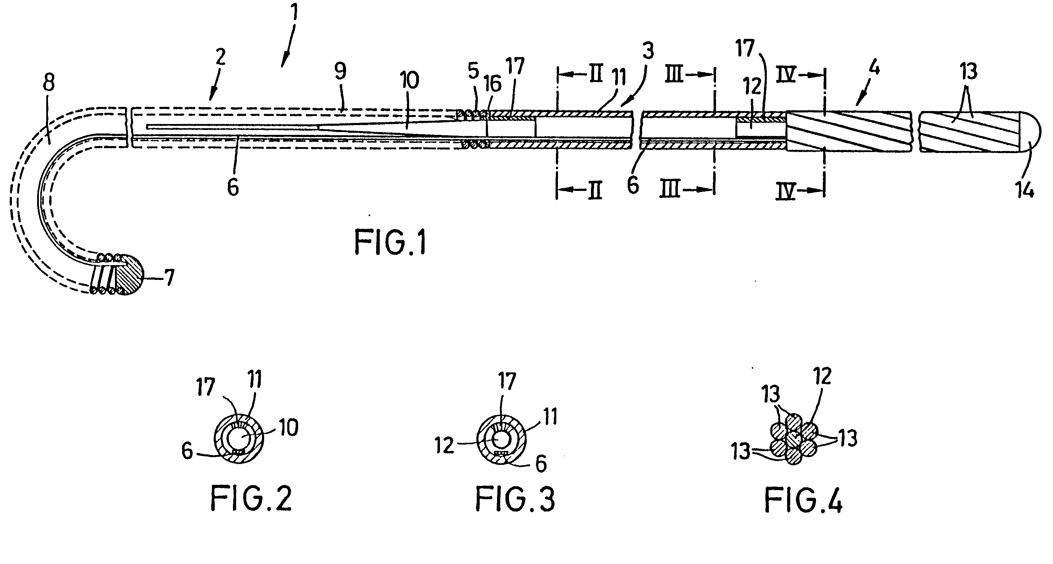

Der Führungsteil 1 gemäß Fig. 1 bis 4 besteht im wesentlichen aus einer weich-flexiblen Spitze 2, einem relativ steifen Schaft 3 und einem flexibel-biegbaren Strangkörper 4, die aus Metall gefertigt und durch Lötung miteinander verbunden sind. Die weich-flexible Spitze 2 ist aus einem mit sehr geringer Steigung schraubenförmig enggewickelten dünnen Draht 5 aufgebaut, in dessen Inneren ein exzentrischer Fangdraht 6 in Form eines flachen Bandes (Fig. 2 und 3) verläuft, dessen äußeres Ende in einen Spitzenkörper 7 eingeschweißt ist, der als grat- und riefenfreier, halbkugelförmig verrundeter Schweißpunkt ausgebildet ist und den Kopf des gewickelten Drahtes 5 bildet, mit dem er ebenfalls verschweißt ist. Die weich-flexible Spitze 2 ist bei 8 J-förmig gekrümmt und in ihrem geraden Teil 9 befindet sich ein gegen den Krümmungsteil 8 verjüngter Stiftkörper 10, dessen stärkeres Ende in den relativ steifen Schaft 3 hineinragt und sowohl mit diesem als auch mit dem Draht 5 der Spitze 2 an den Stellen 16 und 17 fest verlötet ist.

Der relativ steife Schaft 3 hat den gleichen Außendurchmesser wie die weich-flexible Spitze 2 und er besteht aus einem Rohr 11 aus Metall, das verhältnismäßig steif ist, so daß der Schaft 3 zur Führung von Dilatatoren u. dgl. durch Haut, Muskeln und Fascien hindurch geeignet ist. Der Fangdraht 6 erstreckt sich durch den Schaft 3 exzentrisch hindurch und ist mit dem flexibel-biegbaren Strangkörper 4 verbunden, dessen Außendurchmesser demjenigen des Schaftes 3 entspricht.

Während der Schaft 3 eine Länge von etwa 30 cm haben kann, beträgt die Länge des flexibelbiegbaren Strangkörpers 4 mindestens etwa 50 cm. Der flexibel biegbare Strangkörper 4 ist aus einer Seele 12 und diese konzentrisch umgebenden Drähten 13 aufgebaut, die mit starker Steigung verseilt (geschlagen) sind. Die Drähte 13 können rechts oder links geschlagen sein. Zur Erzielung größerer Steifigkeit können mehrere Lagen von Drähten 13 übereinandergewickelt werden. Die Seele 12 steht ein Stück über die geradflächig abgeschnitten Drähte 13 vor und greift in den rohrförmigen Schaft 11 ein, in dem sie befestigt ist. Die Verbindung der Teile 6, 5, 10 und 11 bzw. 4, 6, 11 und 12 geschieht mittels Löt- oder Schweißstellen 17.

## Patentansprüche

1. Führungsteil für einen in einen Körperhohlraum einzuführenden langgestreckten Gegenstand, wie einen Katheterschlauch, einen rohrförmigen Dilatator od. dgl., mit einem drahtförmigen Teil (34), der einen flexibel-biegbaren Strangkörper aufweist der als Litze aus verseilt umeinandergewickelten Drähten ausgebildet ist, dadurch gekennzeichnet, daß zwischen dem flexibel-biegbaren Strangkörper (4) und einer weich-flexiblen Spitze (2) ein relativ steifer Schaft (3) angeordnet ist.

2. Führungsteil nach Anspruch 1, dadurch gekennzeichnet, daß der relativ steife Schaft (3) aus einem Rohr oder einem massiven Runddraht besteht.

3. Führungsteil nach Anspruch 1 oder 2, dadurch gekennzeichnet, daß der flexibel-biegbare Strangkörper (4) länger ist als der relativ steife Schaft (3).

4. Führungsteil nach einem der Ansprüche 1 bis 3, dadurch gekennzeichnet, daß der relativ steife Schaft (3) etwa 30 cm lang, der flexibelbiegbare Strangkörper (4) mindestens etwa 50 cm lang und die Spitze (2) etwa 6 cm lang sind.

5. Führungsteil nach einem der Ansprüche 1 bis 3, dadurch gekennzeichnet, daß der relativ steife Schaft (3) etwa 1 bis 5 cm lang, der flexibelbiegbare Strangkörper (4) etwa 70 bis 80 cm lang und die Spitze (2) etwa 2 bis 5 cm lang sind.

6. Führungsteil nach einem der Ansprüche 1 bis 5, dadurch gekennzeichnet, daß die Drähte (13) der Litze des flexibel-biegbaren Strangkörpers (4) in mindestens einer Lage konzentrisch um eine Seele (12) gewickelt sind.

7. Führungsteil nach einem der Ansprüche 1 bis 6, dadurch gekennzeichnet, daß die weich-flexible Spitze (2) aus einem dünnen Draht (9) besteht, der mit sehr geringer Steigung schraubenförmig enggewickelt ist und im Inneren einen Fangdraht (6) enthält, der einenends mit einem äußeren Spitzenkörper (7) und anderenends mit dem drahtförmigen Teil (3, 4) verbunden ist.

8. Führungsteil nach Anspruch 7, dadurch gekennzeichnet, daß die Spitze (2) gerade oder J-förmig gekrümmt ausgebildet ist.

9. Führungsteil nach einem der Ansprüche 1 bis 8, dadurch gekennzeichnet, daß der drahtförmige Teil (3, 4) und die Spitze (2) aus rostfreiem Metall hergestellt sind.

10. Führungsteil nach Anspruch 9, dadurch gekennzeichnet, daß der drahtförmige Teil (3, 4) mit einer Kunststoffummantelung umgeben ist.

11. Führungsteil nach einem der Ansprüche 1 bis 10, dadurch gekennzeichnet, daß der drahtförmige Teil (3, 4) aus Kunststoff hergestellt ist.

## Claims

1. Guide for an elongated object to be inserted into a body cavity, such as a catheter tube, a tubular dilator or the like, with a wire-like part (3, 4), which has a flexible-bendable extrudate constructed as a strand of wires wound in twisted manner around one another, characterized in that between the flexible-bendable extrudate (4) and a soft flexible tip (2) is arranged a relatively rigid shaft (3).

2. Guide according to claim 1, characterized in that the relatively rigid shaft (3) comprises a tube or a solid wire rod.

3. Guide according to claims 1 or 2, characterized in that the flexible-bendable extrudate (4) is longer than the relatively rigid shaft (3).

4. Guide according to one of the claims 1 to 3, characterized in that the relatively rigid shaft (3) is approximately 30 cm long, the flexible-bendable extrudate (4) at least approximately 50 cm long and the tip (2) approximately 6 cm long.

5. Guide according to one of the claims 1 to 3, characterized in that the relatively rigid shaft (3) is approximately 1 to 5 cm long, the flexible-bendable extrudate (4) approximately 70 to 80 cm long and the tip (2) approximately 2 to 5 cm long.

6. Guide according to one of the claims 1 to 5, characterized in that the wires (13) of the strand of the flexible-bendable extrudate (4) are concentrically wound in at least one layer about a core (12).

7. Guide according to one of the claims 1 to 6, characterized in that the soft flexible tip (2) comprises a thin wire (9), which is helically closely wound with a very limited pitch and contains in the interior a guard wire (6), connected at one end to an outer tip body (7) and at the other to the wire-like part (3, 4).

8. Guide according to claim 7, characterized in that the tip (2) is straight or J-shaped.

9. Guide according to one of the claims 1 to 8, characterized in that the wire-like part (3, 4) and the tip (2) are made from stainless metal.

10. Guide according to claim 9, characterized in that the wire-like part (3, 4) is surrounded by a plastic envelope.

11. Guide according to one of the claims 1 to 10, characterized in that the wire-like part (3, 4) is made from plastic.

## Revendications

1. Elément de guidage pour un objet allongé, par exemple un tuyau de cathéter, un dilatateur tubulaire ou analogues, à introduire dans une cavité corporelle, avec un élément filiforme (3, 4) comportant un corps de boyau souple et flexible réalisé sous la forme d'un cordon souple et flexible de fils toronnés et enroulés les uns sur les autres, caractérisé en ce qu'une tige (3) relativement rigide est disposée entre le corps de boyau souple et flexible (4) et une pointe (2) molle et flexible.

2. Elément de guidage selon la revendication 1, caractérisé en ce que la tige (3) relativement rigide est constituée par un tube ou un fil massif de section circulaire.

3. Elément de guidage selon l'une des revendications 1 ou 2, caractérisé en ce que le corps de boyau (4) souple et flexible est plus long que la tige (3) relativement rigide.

4. Elément de guidage selon l'une des revendications 1 à 3, caractérisé en ce que la tige (3) relativement rigide présente une longueur d'environ 30 cm, que la longueur du corps de boyau (4) souple et flexible est d'au moins 50 cm environ et que la longueur de la pointe (2) est d'environ 6 cm.

5. Elément de guidage selon l'une des revendications 1 à 3, caractérisé en ce que la tige (3) relativement rigide présente une longueur d'environ 1 à 5 cm, que la longueur du corps de boyau (4) souple et flexible est d'environ 70 à 80 cm et que la longueur de la pointe (2) est d'environ 2 à 5 cm.

6. Elément de guidage selon l'une des revendications 1 a 5, caractérisé en ce que les fils (13) du cordon du corps de boyau (4) souple et flexible sont enroulés concentriquement en au moins une couche autour d'une âme (12).

7. Elément de guidage selon l'une des revendications 1 à 6, caractérisé en ce que la pointe (2) molle et flexible est réalisée à partir d'un fil mince (9) qui est enroulé en hélice avec un très petit pas et contient à l'intérieur un fil de protection (6) dont l'une des extrémités est reliée à un corps de pointe (7) extérieur et dont l'autre extrémité est rattachée à l'élément filiforme (3, 4).

8. Elément de guidage selon la revendication 7, caractérisé en ce que la pointe (2) est rectiligne ou courbée en J.

9. Elément de guidage selon l'une des revendications 1 à 8, caractérisé en ce que l'élément filiforme (3, 4) et la pointe (2) sont réalisés à partir d'un métal inoxydable.

10. Elément de guidage selon la revendication 9, caractérisé en ce que l'élément filiforme (3, 4) est muni d'une enveloppe en matière plastique.

11. Elément de guidage selon l'une des revendications 1 à 10, caractérisé en ce que l'élément filiforme (3, 4) est réalisé en matière plastique.

FIG.1

FIG.2

FIG.3

FIG.4

EP 0 200 919 B1